# EUROPEAN PATENT SPECIFICATION

(11) **EP 2 409 649 B1**
(45) Date of publication and mention of the grant of the patent: **05.06.2013**
(21) Application number: 11163289.9
(22) Date of filing: 20.04.2011
(51) Int. Cl.: A61B 8/06, G01S 7/52

(54) **Ultrasonic diagnostic apparatus and method thereof**
Ultraschalldiagnosevorrichtung und Verfahren dafür
Appareil de diagnostic ultrasonique et procédé associé

(30) Priority: 19.07.2010 KR 20100069392
(43) Date of publication of application: 25.01.2012
(73) Proprietor: Samsung Medison Co., Ltd., Nam-myun Hongchun-gun Kangwon do 250-875 (KR)
(72) Inventor: Lee, Jin Yong, Seoul (KR)
(74) Representative: Schmid, Wolfgang

(56) References cited:
- US-A1- 2004 267 127
- US-A1- 2007 055 161
- US-B1- 6 213 945

## Description

### TECHNICAL FIELD

The present disclosure relates to an ultrasonic diagnostic apparatus and a diagnosis method thereof. Particularly, upon ultrasonic diagnosis of an object, the present invention allows a user to easily locate a desired time point and section among the whole information regarding ultrasound images and a variety of measurement data which are obtained over extended use of the ultrasonic diagnosis apparatus.

### BACKGROUND

Ultrasonic diagnostic apparatuses are used in a wide range of applications. For example, ultrasonic diagnostic apparatuses are used in a wide range of medical applications due to non-invasive and non-destructive characteristics thereof. Recently, high performance ultrasonic diagnostic apparatuses have been used to provide two-dimensional or three-dimensional images of internal organs.

Generally, a probe of the ultrasonic diagnostic apparatus includes a transducer for transmitting and receiving wideband ultrasound signals. When the transducer is electrically driven, an ultrasound signal is generated by the transducer and delivered to an object. An ultrasound wave echo signal, which is reflected from the object to the transducer, is transformed into an electrical signal. Then, the transformed electrical signal is amplified and processed to provide ultrasound image data.

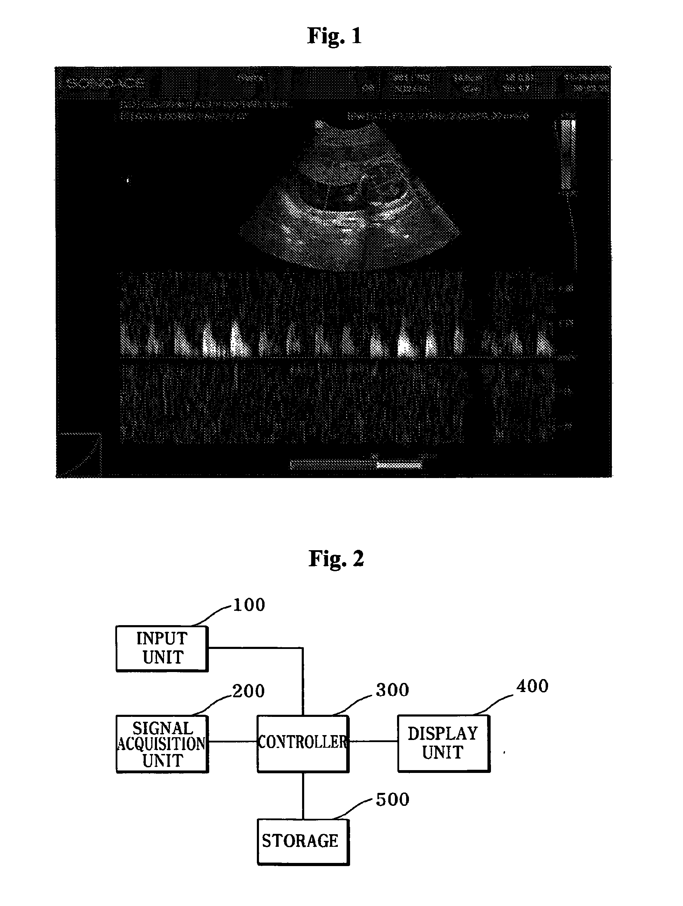

Fig. 1 is a view explaining an image display method of a display unit of a conventional ultrasonic diagnostic apparatus. In the related art, as shown in Fig. 1, a B-mode (brightness mode) image which displays magnitude of ultrasound echo signals reflected from an object with brightness is displayed for example on an upper side of a screen. Measuring information or biological information, such as a blood flow rate at a location that corresponds to a section (dotted line) specified by a user in the B-mode image, is displayed on a lower side below the B-mode image. Accordingly, the measuring information for the corresponding specified section can be displayed with time.

In addition, in the conventional ultrasonic diagnostic apparatus, a range bar is also provided at the lower side of the screen in Fig. 1 to indicate to which section in the whole diagnosis period the current time point belongs. Accordingly, through the range bar, a user can recognize the section in the whole diagnosis period, to which the currently displayed image belongs.

However, according to the conventional ultrasonic diagnostic apparatus, a user can only recognize the section in the whole diagnosis period by observing the range bar displayed on the display screen, but in fact, the user cannot easily check a section of interest (e.g., a section in which blood flow rate excessively increases). That is, since the display screen displays only an image at a current time point or an image near a time point selected in the range bar, a user cannot easily check or identify a section in which abnormal event has occurred. Thus, in order to check such a section, the user must slowly examine the whole period in detail using a track ball or the like provided to the apparatus so as to reach the section of interest.
The preferred embodiments described in the US 6,213,945 B1 provide a method and system for generating a graphical vascular report. Unlike prior vascular calculation packages that generate a report listing the results along with the assigned vessel location name, the use of a graphical vascular report provides a visual roadmap of the vascular system under study, incorporating calculations and results in an at-a-glance display. These preferred embodiments make vascular calculations more meaningful to a wider number of physicians and promote the use of vascular calculations to a wider number of disciplines. Additionally, these preferred embodiments improve the presentation of outgoing reports by directly associating results to target areas. Further, the graphical vascular reports of these preferred embodiments are easy to create and are easily duplicated and stored.

### SUMMARY

To improve the art and address the above, the present disclosure provides an ultrasonic diagnostic apparatus and a diagnosis method thereof, which allow a user to easily locate a desired time point and section among the whole information regarding ultrasound images and a variety of measurement data which are obtained over extended use of the ultrasonic diagnosis apparatus.

In accordance with one aspect, an ultrasonic diagnostic apparatus: displaying an ultrasound image obtained from an object on a first region of a display unit, and displaying image information regarding measurement data with respect to a desired section of the ultrasound image on a second region of the display unit, wherein the image information displayed on the second region includes an image map in which overall measurement data obtained at respective diagnosis points are accumulated in a time-series manner. Image magnification of the image map is automatically controlled with respect to a time period of data accumulation according to a total amount of the accumulated measurement data.

The apparatus may allow a user to search the measurement data and select a desired time point using the image map, and detailed measurement data at the selected time point may be displayed on a third region of the display unit.

The measurement data may include blood flow rate.

The apparatus may include: a signal acquisition unit configured to receive an ultrasound signal reflected from an object; the display unit; an input unit; a storage; and a controller configured to control the apparatus to create an ultrasound image using the ultrasound signal, to provide the ultrasound image and image information to the display unit, to display detailed measurement data corresponding to a time point in the image map selected through the input unit by a user on the third region of the display unit, and to store all or part of the measurement data in the storage.

The apparatus may allow a user to search the measurement data and select a desired time point using the image map, and the measurement data corresponding to the selected time section may be stored in the storage.

In accordance with another aspect, a diagnosis method of an ultrasonic diagnostic apparatus which displays an ultrasound image obtained from an object on a first region of a display unit, comprises: obtaining measurement data that corresponds to a specified section of the ultrasound image selected by a user and displaying, on a second region of the display unit, an image map in which the obtained measurement data are accumulated in a time-series manner; receiving a desired time point in the image map selected by a user; and displaying, on a third region of the display unit, measurement data corresponding to the selected time point.

The method may further include: receiving a desired time section selected through the image map by a user; and storing the measurement data corresponding to the selected time section in a storage.

### BRIEF DESCRIPTION OF THE DRAWINGS

The figures depict one or more implementations in accordance with the present disclosure, by way of example only, not by way of limitation. In the figures, like reference numerals refer to the same or similar elements.

Fig. 1 is a view explaining an image display method of a display unit of a conventional ultrasonic diagnostic apparatus;

Fig. 2 is a view of an ultrasonic diagnostic apparatus according to an exemplary embodiment of the present disclosure;

Fig. 3 is a view explaining an image display method of a display unit of the ultrasonic diagnostic apparatus of the embodiment of the present disclosure; and

Fig. 4 is a view explaining variation in image magnification with respect to time period in an image map of the apparatus according to the embodiment of the present disclosure.

### DETAILED DESCRIPTION

Exemplary embodiments of the present disclosure will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the present disclosure into account and can be changed according to the custom or intention of users or operators. Therefore, the terms should be defined according to the overall disclosures set forth herein.

Fig. 2 is a view of an ultrasonic diagnostic apparatus according to an exemplary embodiment of the present disclosure.

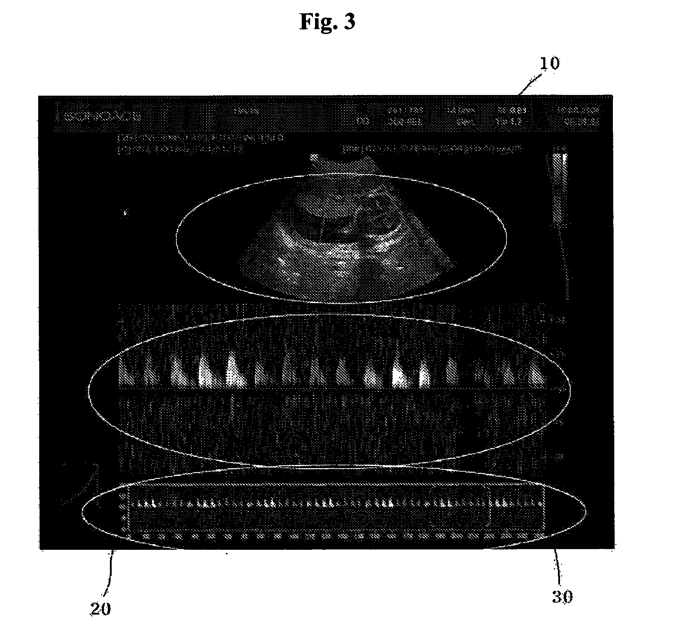

Referring to Fig. 2, the ultrasonic diagnostic apparatus of this embodiment includes an input unit 100; a signal acquisition unit 200 receiving an ultrasound signal reflected from an object; a display unit 400; a storage 500; and a controller 300. The controller 300 controls the apparatus to create an ultrasound image using the ultrasound signal, to provide the ultrasound image and image information regarding measurement data to the display unit 400, to display detailed measurement data, which corresponds to a time point in an image map selected through the input unit 100 by a user, on a third region 30 (see Fig. 3) of the display unit 400, and to store all or part of the measurement data in the storage 500.

The apparatus according to the embodiment constructed as described above will now be described with reference to Figs. 2 to 4.

First, the signal acquisition unit 200 transmits an ultrasound signal to an object and receives the ultrasound signal reflected from the object to create an ultrasound image.

The controller 300 creates the ultrasound image using the ultrasound signal sent from the signal acquisition unit, and provides the ultrasound image to the display unit 400. The controller 300 allows the ultrasound image to be displayed in a B-mode (brightness-mode) on a first region 10 at an upper side of a screen of the display unit 400. Here, the B-mode means a diagnostic mode in which the magnitude of an ultrasound echo signal reflected from the object is displayed based upon brightness on the screen. It should be understood that the ultrasound image can be displayed on the screen in a variety of other modes according to embodiments.

Further, the controller 300 creates image information regarding measurement data that corresponds to a specified section (dotted line section of reference numeral 10 in Fig. 3) of the ultrasound image selected through the input unit 100 by a user, and provides the image information to the display unit 400. Here, the controller 300 allows the image information to be displayed in a PW-mode (pulse wave mode) on a second region 20 at a lower side of the screen of the display unit 400. Actually, the PW-mode means a diagnostic mode in which a blood flow rate with respect to a predetermined specified section of the object is displayed as a graphical image of time-blood flow rate. As the input unit 100, all of available user interfaces may be employed, and particularly the input unit may also be formed in a touch-screen form.

However, when displaying the image information on the second region 20, the present embodiment is different from the conventional case. That is, since the conventional PW-mode illustrates information on the blood flow rate at the respective diagnosis points as an image pattern that changes over time, as shown in the middle of Fig. 1 or in the third region 30 of Fig. 3, the information on the blood flow rate at time points that have passed cannot be displayed, when a certain period of time (few seconds to tens of seconds) elapses. However, according to the present embodiment, the image information displayed on the second region 20 includes an image map which accumulates all information regarding the blood flow rate, i.e. all time-series measurement data, obtained at the respective diagnosis points. Accordingly, when observing the image map displayed on the second region 20, the information regarding the blood flow rate can be easily and effectively checked even after the diagnosis point has considerably elapsed. Thereby a user can recognize a time point when an abnormal event such as an excessive increase in blood flow rate has occurred.

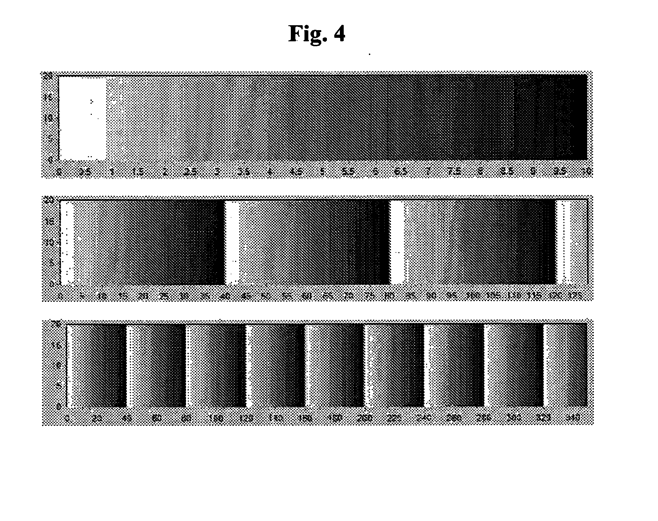

In this embodiment, in order to allow the blood flow rate information with respect to an entire diagnosis time period to be included in the image map displayed on the second region 20, the controller 300 enables an image magnification of the measurement data with respect to a diagnosis time period (time period of data accumulation) to be automatically regulated based on the total amount of measurement data (blood flow rate information) accumulated over time when forming the image map. Namely, if only a short period of time, for example 10 seconds, passes after starting diagnosis, the image is allowed to be displayed in a relatively large scale as shown in the first figure of Fig. 4, and, as the time continuously passes, the image magnification of the measurement data with respect to the time period is automatically regulated such that it gradually decreases. According to such a display method, even though a long period of time has elapsed after starting the diagnosis, overall measurement data are included in the image map and can be displayed. Accordingly, even if a displayed image becomes reduced in size over time after starting the diagnosis, a user can easily locate and determine a time point where an abnormal event has occurred.

In the meantime, during diagnosis, the controller 300 allows a PW-mode image with respect to information regarding a blood flow rate at a time point of an abnormal event to be displayed on the third region 30.

In addition, when a user searches for the blood flow rate information using the image map and selects the time point where the abnormal event has occurred after completion of diagnosis or even during diagnosis, the controller 300 allows detailed measurement data (i.e. blood flow rate information) at the selected point to be displayed on the third region 30 in the middle of the display unit 400. Here, the image displayed on the third region 30 corresponds to a section defined by brackets [˙] of the second region 20 of Fig. 3. Alternatively, in certain systems, it may be configured such that a predetermined section after and before a time point selected by a user is displayed as an image on the third region, or otherwise it may be configured to allow a user to directly set a start point and an end point of a section.

Further, the controller 300 may allow all or a portion of measurement data such as blood flow rate information or the like to be stored in the storage 500. Particularly, when a user selects a specified time section using the image map, the controller 300 may allow the measurement data, such as blood flow rate information, and/or an ultrasound image, which correspond to the selected time section, to be stored in the storage 500.

Although the above embodiment refers to the configuration wherein the PW-mode image map including blood flow rate information is displayed on the second region 20, it should be understood that in certain embodiments an image map may be displayed in a M-mode (motion-mode) or in a CW-mode (continuous wave mode), which shows motion of an object in a periodic manner, and other diverse modes are also applicable.

Although a controller can be divided into a signal processor for processing signals and a control unit serving as a processor in certain embodiments, it should be understood that the controller according to the present embodiment encompasses such a modification of the controller. In addition, the storage 500 of the present embodiment means all kinds of memories or storage media generally including a volatile memory, a non-volatile memory, an internal memory, an external memory, and the like.

As such, according to the embodiment, the apparatus allows a user to effectively locate and determine a section of interest, such as a section where an abnormal event (e.g., an excessive increase in blood flow rate) has occurred, using an image map which accumulates measurement data obtained during diagnosis. Further, when the section of interest in the image map is selected by a user, the apparatus allows the user to check corresponding detailed measurement data. Furthermore, the apparatus allows required or useful information to be stored and effectively utilized in the future.

In the ultrasonic diagnostic apparatus and method, a user can easily locate a desired time point and section among the whole information regarding ultrasound images and a variety of measurement data which are obtained over extended use of the ultrasonic diagnosis apparatus, and data corresponding to the desired section selected by the user can be stored for future use.

While the foregoing has described what are considered to be the best mode and/or other examples, it is understood that various modifications may be made therein and that the subject matter disclosed herein may be implemented in a variety of different ways, and that the present disclosure may be applied to numerous applications, only some of which have been described herein. The following claims should be considered to cover all applications, modifications and variations within the true scope of the present disclosure.

## Claims

1. An ultrasonic diagnostic apparatus including a display unit (400),
the ultrasonic diagnostic apparatus being configured to display an ultrasound image obtained from an object on a first region (10) of the display unit (400), and display image information regarding measurement data with respect to a desired section of the ultrasound image on a second region (20) of the display unit (400),
wherein the image information displayed on the second region (20) comprises an image map in which overall measurement data obtained at respective diagnosis points are accumulated in a tim-series manner,
**characterized in that** the apparatus is configured such that an image magnification the image map is automatically regulated with respect to a time period of data accumulation according to a total amount of the accumulated measurement data.

2. The apparatus of claim 1, **characterized in that** the apparatus allows a user to search the measurement data and select a desired time point using the image map, and detailed measurement data at the selected time point is displayed on a third region (30) of the display unit (400).

3. The apparatus of claim 1, **characterized in that** the measurement data includes blood flow rate.

4. The apparatus of claim 1, further comprising:
a signal acquisition unit (200) configured to receive an ultrasound signal reflected from an object;
an input unit (100);
a storage (500); and
a controller (300) configured to control the apparatus to create an ultrasound image using the ultrasound signal, to provide the ultrasound image and image information to the display unit (400), to display detailed measurement data corresponding to a time point in the image map selected through the input unit (100) by a user on a third region (30) of the display unit (400), and to store all or part of the measurement data in the storage (500).

5. The apparatus of claim 5, **characterized in that** the apparatus allows a user to search the measurement data and select a desired time section using the image map, and the measurement data corresponding to the selected time section is stored in the storage (500).

6. A diagnosis method of an ultrasonic diagnostic apparatus, which displays an ultrasound image obtained from an object on a first region (10) of a display unit (400), comprising:
obtaining measurement data that corresponds to a specified section of the ultrasound image selected by a user and displaying, on a second region (20) of the display unit (400), an image map in which the obtained measurement data are accumulated in a time-series manner;
receiving a desired time point in the image map selected by a user; and
displaying, on a third region (30) of the display unit (400), measurement data corresponding to the selected time point,
**characterized in that** an image magnification of the image map is automatically regulated with respect to a time period of data accumulation according to a total amount of the accumulated measurement data.

7. The method of claim 6, **characterized in that** the measurement data comprises blood flow rate.

8. The method of claim 6 or 7, further comprising:
receiving a desired time section selected through the image map by a user; and
storing the measurement data corresponding to the selected time section in a storage (500).

## Patentansprüche

1. Ultraschalldiagnosevorrichtung mit einer Anzeigeeinheit (400),
wobei die Ultraschafldiagnosevorrichtung dafür vorgesehen ist, ein von einem Objekt erlangtes Ultraschallbild auf einem ersten Bereich (10) der Anzeigeeinheit (400) anzuzeigen und Bildinformationen, die sich auf Messdaten bezüglich eines gewünschten Abschnitts des Ultraschallbilds auf einem zweiten Bereich (20) der Anzeigeeinheit (400) beziehen, anzuzeigen,
wobei die in dem zweiten Bereich (20) angezeigten Bildinformationen eine Bildkarte bzw. eine Textur aufweisen, in der in jeweiligen Diagnosepunkten erlangte Messdaten in einer zeitlichen Abfolge angesammelt sind,
**dadurch gekennzeichnet, dass**
die Vorrichtung so ausgebildet ist, dass eine Bildvergräßerung der Bildkarte im Hinblick auf eine Zeitdauer der Datenansammlung gemäß einer Gesamtmenge der angesammelten Messdaten automatisch geregelt wird.

2. Vorrichtung nach Anspruch 1,
**dadurch gekennzeichnet, dass**
die Vorrichtung es einem Benutzer erlaubt, die Messdaten zu suchen und unter Verwendung der Bildkarte einen gewünschten Zeitpunkt auszuwählen, und dass detaillierte Messdaten zu dem ausgewählten Zeitpunkt in einem dritten Bereich (30) der Anzeigeeinheit (400) angezeigt werden.

3. Vorrichtung nach Anspruch 1,
**dadurch gekennzeichnet, dass**
die Messdaten einen Blutdurchflussstrom beinhalten.

4. Vorrichtung nach Anspruch 1, welche des Weiteren Folgendes aufweist:
eine Signalerlangungseinheit (200), die in der Lage ist, ein von einem Objekt reflektiertes Ultraschallsignal zu empfangen;
eine Eingabeeinheit (100);
einen Speicher (500); und
eine Steuereinheit (300), weich dafür vorgesehen ist, die Vorrichtung zu steuern, um unter Verwendung des Ultraschallsignals ein Ultraschallbild zu erzeugen, das Ultraschallbild und Bildinformationen an die Anzeigeeinheit (400) zu liefern, detaillierte Messdaten, die einem Zeitpunkt in der Bildkarte entsprechen, die durch die Eingabeeinheit (100) von einem Benutzer in einem dritten Bereich (30) der Anzeigeeinheit (400) ausgewählt wurde, anzuzeigen, und alle oder einen Teil der Messdaten in dem Speicher (500) zu speichern.

5. Vorrichtung nach Anspruch 4,
**dadurch gekennzeichnet, dass**
die Vorrichtung es einem Benutzer ermöglicht, die Messdaten zu suchen und einen gewünschten Zeitraum unter Verwendung der Bildkarte auszuwähten, und dass die dem ausgewählten Zeitraum entsprechenden Messdaten in dem Speicher (500) gespeichert werden.

6. Diagnoseverfahren für eine Uttraschalldiagnosevorrichtung, weiche ein von einem Objekt erhaltenes Ultraschallbild auf einem ersten Bereich (10) einer Anzeigeeinheit (400) anzeigt, welches folgendes aufweist:
Erlangen von Messdaten, die einem bestimmten, von einem Benutzer ausgewählten Bereich des Ultrischällbilds entsprechen, und Anzeigen einer Text- bzw. Bildkarte, in der die eriangten Messdaten in einer zeitlichen Abfolge angesammelt sind, auf einem zweiten Bereich (20) der Anzeigeeinheit (400);
Empfangen eines gewünschten Zeitpunkts in der Bildkarte, die von einem Benutzer ausgewählt wurde; und
Anzeigen von Messdaten, die dem gewünschten Zeitpunkt entsprechen, auf einen dritten Bereich (30) der Anzeigeeinheit (400),
**dadurch gekennzeichnet, dass**
eine Bildvergrößerung der Bildkarte im Hinblick auf eine Zeitdauer der Datenansammlung gemäß einer Gesamtmenge der angesammelten Messdaten automatisch geregelt wird.

7. Verfahren nach Anspruch 6,
**dadurch gekennzeichnet, dass**
die Messdaten einen Blutdurchflussstrom beinhalten.

8. Verfahren nach Anspruch 6 oder 7, welches des Weiteren Folgendes aufweist:
Empfangen eines gewünschten Zeitabschnitts, der von einem Benutzer durch die Bildkarte ausgewählt wurde; und
Speichern der Messdaten, die dem gewünschten Zeitabschnitt entsprechen, in einem Speicher (500).

## Revendications

1. Appareil ultrasonique de diagnostic comportant une unité d'affichage (400),
l'appareil ultrasonique de diagnostic étant configuré pour afficher une image ultrasonique obtenue à partir d'un objet, dans une première zone (10) de l'unité d'affichage (400) et pour afficher des informations d'image relatives à des données de mesures en relation avec la zone choisie de l'image ultrasonique, dans une deuxième zone (20) de l'unité d'affichage (400),
dans lequel l'information d'image affichée dans la deuxième zone (20) comporte une carte image dans laquelle des données de mesures totales obtenues respectivement en divers points d'analyse, sont accumulées selon une manière « time-series »,
**caractérisé en ce que** l'appareil est configuré de telle manière qu'une amiplification d'image de la carte image est automatiquement régulée en fonction de l'intervalle de temps requis pour l'accumulation de données selon la somme totale de données de mesures accumulées.

2. Appareil selon la revendication 1, **caractérisé en ce que** l'appareil permet à un utilisateur de rechercher les données de mesures et de sélectionner un point temps souhaité en utilisant la carte image et les données de mesures détaillées de ce point temps sélectionné sont affichées dans une troisième zone (30) de l'unité d'affichage (400).

3. Appareil selon la revendication 1, **caractérisé en ce que** les données de mesures contiennent le rythme du flux sanguin.

4. Appareil selon la revendication 1, comprenant en outre :
une unité d'acquisition de signal (200) configurée pour recevoir un signal ultrasonique réfléchi par un objet ;
une unité d'entrée (100) ;
une unité de stockage (500) ; et
un contrôfeur (300) agencé pour commander l'appareil pour créer une image ultrasonique utilisant un signal ultrasonique, pour fournir une image ultrasonique et des données ultrasoniques à l'unité d'affichage (400), pour afficher des mesures détaillées correspondant à un point temps de la carte image sélectionné par l'unité d'entrée (100) par un utilisateur dans la troisième zone (30) de l'unité d'affichage (400) et pour stocker toutes les parties des données de mesures dans l'unité de stockage (500).

5. Appareil selon la revendication 4, **caractérisé en ce que** l'appareil permet à un utilisateur de rechercher les données de mesures et de sélectionner un intervalle de temps souhaité en utilisant la carte image, et les donnés de mesures correspondant à l'intervalle de temps sélectionné sont stockées dans l'unité de stockage (500).

6. Procédé de diagnostic d'un appareil de diagnostic ultrasonique, qui affiche une image ultrasonique d'un objet dans une première zone (10) d'une unité d'affichage (400) comprenant :
l'obtention de données de mesures qui correspondent à la zone spécifique de l'image ultrasonique sélectionnée par un utilisateur et l'affichage dans une deuxième zone (20) de l'unité d'affichage (400) d'une carte image dans laquelle sont accumulées toutes les données des mesures selon la manière « time-series » ;
la réception d'un point temps souhaité de la carte image, sélectionné par un utilisateur; et
l'affichage dans une troisième zone (30) de l'unité d'affichage (400) de données de mesures correspondant au point temps sélectionné,
**caractérisé en ce qu'**une amplifcation d'image de la carte image est automatiquement régulée en fonction de l'intervalle de temps requis pour l'accumulation de données selon la somme totale de données de mesures accumulées

7. Procédé selon la revendication 6, **caractérisé en ce que** les données de mesures contiennent le rythme du flux sanguin.

8. Procédé selon les revendications 6 ou 7, comprenant en outre :
la réception d'un intervalle de temps souhaité sélectionné au moyen de la carte image par un utilisateur, et
le stockage des données de mesures correspondant à l'intervalle de temps sélectionné dans une unité de stockage (500).
